# EUROPÄISCHE PATENTSCHRIFT

(11) **EP 3 694 599 B1**
(45) Veröffentlichungstag und Bekanntmachung des Hinweises auf die Patenterteilung: **10.01.2024**
(21) Anmeldenummer: 18795943.2
(22) Anmeldetag: 11.10.2018
(51) Int. Cl.: A61N 1/30

(54) **IN EINER HAND HALTBARES GERÄT ZUR ELEKTRISCH UNTERSTÜTZTEN HAUTBEHANDLUNG, ZUSATZTEIL FÜR DIESES GERÄT UND BLISTER FÜR DIESES ZUSATZTEIL**
DEVICE THAT CAN BE HELD ON ONE HAND FOR ELECTRICALLY ASSISTED SKIN TREATMENT, ADDITIONAL PART FOR SAID DEVICE AND BLISTER FOR SAID ADDITIONAL PART
APPAREIL PORTATIF À LA MAIN POUR LE TRAITEMENT ÉLECTRIQUEMENT ASSISTÉ DE LA PEAU, ACCESSOIRE POUR CET APPAREIL ET BLISTER POUR CET ACCESSOIRE

(30) Priorität: 12.10.2017 DE 102017123809
(43) Veröffentlichungstag der Anmeldung: 19.08.2020
(60) Teilanmeldung: 23212813.2
(73) Patentinhaber: Swiss Spa System Ltd., Hong Kong (CN)
(72) Erfinder: GIMELLI, Bruno, 3052 Zollikofen (CH); DOYLE, James N., Westlake Ohio 44145 (US)
(74) Vertreter: Rauch, Udo
(86) Internationale Anmeldenummer: PCT/EP2018/077796
(87) Internationale Veröffentlichungsnummer: WO 2019/073001

(56) Entgegenhaltungen:
- WO-A2-03/018116
- DE-A1-102012 009 514
- DE-U- 1 805 711
- DE-U1- 9 017 597

## Beschreibung

Die Erfindung bezieht sich auf ein in einer Hand haltbares Gerät zur elektrisch unterstützten Hautbehandlung, aufweisend:
Eine Hautelektrode, die auf den zu behandelnden Hautbereich aufsetzbar ist,
eine Handelektrode an der Außenseite des Gerätes, die in Kontakt zur Hand steht, wenn das Gerät vom Nutzer beim Gebrauch in der Hand gehalten wird,
eine elektrische Energiequelle in dem Gerät, deren Pole beim Betrieb des Gerätes mit den Elektroden in einer elektrischen Verbindung stehen,
wobei sich vor der Hautelektrode ein mit einer Wirksubstanz getränktes, saugfähiges Trägermaterial befindet.

Ein derartiges Gerät ist z. B. in der WO 2005/087308 A1 oder in der WO 00/30711 beschrieben. Während in der älteren Anmeldung WO 00/30711 A1 die Hautelektrode noch einheitlich mit einer Basis ausgeführt ist, wird gemäß der WO 2005/087308 A1 vorgeschlagen, die Hautelektrode in Form einer auswechselbaren Kappe auszubilden, wobei unterschiedlich geformte Kappen für die Behandlung spezifischer Hautbereiche bereit gestellt werden. Die Geräte gemäß den beiden Dokumenten sehen vor, die Einwirkung von Wirksubstanzen in einer Hautcreme dadurch zu verbessern, dass über die Hautelektrode ein Strom in den Körper mit negativer bzw. positiver Polarität geleitet wird. Bei den Wirksubstanzen kann es sich um kosmetisch aber auch medizinisch wirksame Substanzen z. B. für die Wundheilbehandlung handeln. Mit der einen Polarität werden die Wirksubstanzen der Creme in die Haut geleitet, während bei der anderen Polarität Verschmutzungen aus der Haut in eine Reinigungscreme wandern. In beiden Fällen muss vor Anwendung des Gerätes zunächst eine entsprechende Creme aufgetragen werden.

Der Inhalt der beiden Schriften wird, soweit er den mechanischen und elektrischen Aufbau des Geräts betrifft, auch zum Inhalt der vorliegenden Anmeldung gemacht.

Problematisch dabei ist die richtige Dosierung. Bei einer zu geringen Dosierung wird zu wenig Wirksubstanz in die Haut eingebracht bzw. Verschmutzungen entfernt, eine zu hohe Dosierung kann nicht vollständig ausgenutzt werden, so dass die Creme nach der Hautbehandlung teilweise ungenutzt wieder entfernt werden muss. Darüber hinaus müssen, nachdem die Creme auf die Haut aufgetragen worden ist, zunächst die Hände gereinigt werden, damit das Hautbehandlungsgerät sicher gehalten werden kann.

In der DE 1 805 711 U1 wie auch in der DE 90 17 597 U1 wurde schon vorgeschlagen, einen Schwamm, der mit einer Wirksubstanz getränkt ist, vor der Elektrode zu platzieren. Dazu läuft ein nach innen gebogener Kragen um eine plattenförmige Elektrode, der einen mit einer Wirksubstanz getränkten Schwamm aufnimmt. Aber auch hier besteht das Problem, dass der Schwamm zunächst getränkt werden muss und dann auf die Elektrode platziert werden muss, wobei die Hände mit der Wirksubstanz benetzt werden, die zunächst abgewaschen werden muss, damit anschließend das Gerät sicher gehalten werden kann.

Die WO 03/018116 A2 zeigt ein Hautbehandlungsgerät mit einer Elektrode, auf deren konvex gekrümmter Oberfläche ein Wirkstoffpad befestigt wird. Dazu ist ein ringförmiger, offener Elektrodendeckel vorgesehen, der auf das Gehäuse des Gerätes geschraubt wird, wodurch ein nach innen weisender Flansch des Deckels den Rand des Wirkstoffpads auf der Elektrode festklemmt. Bei dem Wirkstoffpad handelt es sich um ein Einwegpad, das in einem Einfach-Blister luftdicht verpackt ist.

Ein ähnliches Hautbehandlungsgerät ist in DE 9 017 597 U offenbart.

Die Erfindung beruht somit auf der Aufgabe, die Applikation einer Creme zu vereinfachen.

Die Erfindung sieht dazu vor, dass das Gerät ein Zusatzteil aufweist, das aus einem Ring besteht, der auf das Gerät aufsteckbar ist und an dem das mit einer Wirksubstanz getränkte, saugfähige Trägermaterial befestigt ist, wobei das Trägermaterial bei einem aufgesteckten Ring über die Hautelektrode verläuft. Der Ring ist in Form eines Ovals mit zwei Längsabschnitten ausgeführt, die über Bögen miteinander verbunden sind. Das Trägermaterial ist an den Längsabschnitten befestigt.

Ein Ring mit einem mit einer Wirksubstanz getränkten Trägermaterial, das mit einer Wirksubstanz in einer genau dosierten Menge getränkt ist, wird dem Benutzer in geeigneter Weise zur Verfügung gestellt. Dieser kann, ohne dass die Hände mit einer Creme bzw. der darin enthaltenden Wirksubstanz benetzt werden, den Ring erfassen und auf das Gerät aufstecken. Damit gelangt die Creme mit der Wirksubstanz vor den Kopf der Kappe, so dass unmittelbar danach mit der Hautbehandlung begonnen werden kann.

Eine Ausführung des Trägermaterials sieht vor, dass es aus einem zwei Enden und einem dazwischen liegenden Zwischenabschnitt aufweisenden Streifen besteht, dessen Enden an dem Ring befestigt sind und dessen Zwischenabschnitt bei einem aufgesteckten Ring über die Hautelektrode verläuft.

Vorzugsweise ist der Ring an die Kontur des Gerätes angepasst, so dass der aufgesteckte Ring das Gerät möglichst spielfrei umschließt. Weiterhin ist der Kopf des Gerätes sich nach oben verjüngend ausgeführt, so dass der Ring über den Kopf auf das Gerät aufgesetzt werden kann, bis er einen Bereich des Gerätes erreicht, der in seiner Außenkontur der Innenkontur des Ringes entspricht.

Bei der Bewegung des Gerätes über die Haut entstehen Zugkräfte auf das Trägermaterial, daher ist es notwendig, den Ring gegen ein Abziehen zu sichern. Die Erfindung sieht daher weiterhin vor, dass der Ring wenigstens eine nach innen gerichtete Rastnase aufweist, die eine Haltekante am Gerät hintergreift.

Bei einer Ausführung, bei der
die Hautelektrode die Form einer mit einer Basis verbundenen Kappe besitzt, wobei die Kappe einen basisnahen Fußbereich und einen auf die zu behandelnde Haut aufsetzbaren Kopfbereich besitzt,
die Handelektrode an der Außenseite der Basis, die in Kontakt zur Hand steht, wenn das Gerät vom Nutzer beim Gebrauch in der Hand gehalten wird, ausgebildet ist und
die elektrische Energiequelle in der Basis angeordnet ist,
   sieht die Erfindung vor,
dass das Trägermaterial bei einem aufgesteckten Ring über den Kopf der Kappe verläuft.

Der Ring umschließt den Fußbereich der Kappe möglichst passgenau, so dass die Unterkante der Kappe die Haltekante für ein oder mehrere nach innen gerichtete Rastnasen am Ring bildet.

Bei einer Ausführung, bei der die Kappe auf einen Sockel an der Basis aufgesteckt ist, befindet sich zwischen der Unterkante der Kappe und einer Stufe an der Basis ein Spalt, in den die Rastnase eingreift.

Da dieser Spalt nicht sehr tief und nicht sehr breit sein soll, darf die Rastnase nicht weit über die innere Mantelfläche des Ringes hinausweisen. Um der Rastnase trotzdem eine gute Flexibilität zu geben, ist vorgesehen, dass der Ring an der Innenseite eine Quernut aufweist, an deren Boden die Rastnase angeformt ist.

Vorzugsweise sind wenigstens zwei Rastnasen vorgesehen, die sich am Ring gegenüberliegen.

Das Trägermaterial hat vorzugsweise die Form eines Streifens und die Hautelektrode einen länglichen Kopf mit einem halbzylindrischen oberen Abschluss, über den der Streifen verläuft.

Dazu ist der Ring in Form eines Ovals mit zwei Längsabschnitten ausgeführt, die über Bögen miteinander verbunden sind, wobei der Streifen an den Längsabschnitten befestigt ist. Die beiden Bögen dienen als Griffe, mit denen der Ring erfasst werden kann, ohne dass die Finger mit dem Trägermaterial in Berührung kommen.

Das Trägermaterial kann aus den verschiedensten Materialien bestehen. Denkbar wäre ein saugfähiges Gewebe, ein Schwamm oder ein Filz.

Die Erfindung hat weiterhin den Vorteil, dass der Ring als Zusatzteil ausgeformt werden kann, der auch bei schon auf dem am Markt vorhandenen Geräten genutzt werden kann.

Demnach besteht das Zusatzteil aus einem Ring, der auf ein Hautbehandlungsgerät aufsteckbar ist und an den ein mit einer Wirksubstanz getränktes saugfähiges Trägermaterial befestigt ist. Wie schon erläutert, besitzt dieser Ring eine ovale Form mit zwei Längsabschnitten, die über Bögen miteinander verbunden sind, wobei das Trägermaterial aus einem Streifen besteht, der sich von einem Längsabschnitt zum anderen Längsabschnitt erstreckt. Der Ring besitzt an der Innenseite der Längsabschnitte jeweils mindestens eine Rastnase, die über die innere Mantelfläche des Ringes hervorsteht. Um deren ausreichende Biegbarkeit zu gewährleisten, ist vorgesehen, dass die Rastnasen in Quernuten am Ring geordnet sind.

Damit die Wirksubstanz vor ihrem Gebrauch nicht verdampft oder oxidiert, wird das Zusatzteil in einer hermetisch geschlossenen Verpackung vakuumdicht aufbewahrt, die erst bei Bedarf geöffnet wird. Eine solche Verpackung ermöglicht es, eine Creme mit Wirksubstanzen, aber ohne Konservierungsmittel, zu verwenden.

Eine Form der Verpackung sind sogenannte Blister. Die Zusatzteile werden in solchen Blistern zur Verfügung gestellt, wobei der Blister aus einer Schale mit einer Folienabdeckung besteht. In der Schale befindet sich eine umlaufende Vertiefung zur Aufnahme des Ringes, wobei der Ring so in die Vertiefung eingelegt ist, dass der Streifen zum Schalenboden weist und sein mittlerer Teil in einem Bogen durch den Ring verläuft.

Im Folgenden soll anhand eines Ausführungsbeispiels die Erfindung näher erläutert werden. Dazu zeigen die
- Fig. 1: ein typisches Hautbehandlungsgerät, bei dem die Erfindung zum Einsatz kommt,
- Fig. 2: eine perspektivische Ansicht eines Ringes mit einem daran befestigten Trägermaterial,
- Fig. 3: einen Blister mit eingelegten Ringen,
- Fig. 4: einen Querschnitt durch den Ring, bei dem das an dem Ring befestigte Trägermaterial zum Einlegen in den Blister gefaltet ist,
- Fig. 5: einen Ring, der auf das Gerät aufgesteckt wird und
- Fig. 6: einen Teilquerschnitt durch das Gerät mit einem aufgesteckten Ring, wobei eine Rastnase am Ring in einen Spalt am Gerät eingreift.

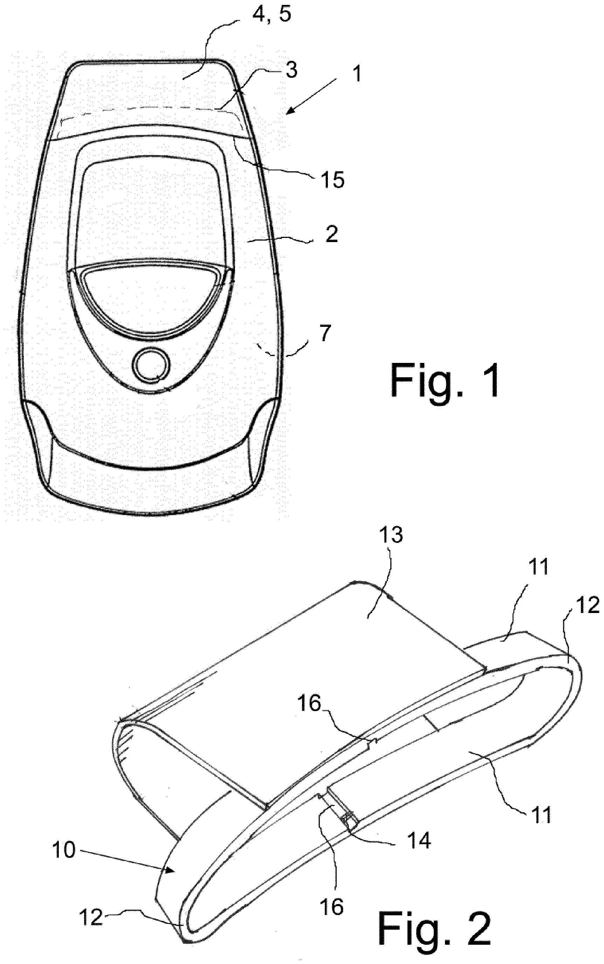

Zunächst wird auf die Fig. 1 Bezug genommen.

Fig. 1 zeigt ein typisches Hautbehandlungsgerät 1 mit einer Basis 2, die in der Hand gehalten werden kann, wobei die Basis 2 an ihrem oberen Ende einen Sockel 3 (strichpunktiert dargestellt, da verdeckt) aufweist, in dem eine Hautelektrode 4 in Form einer Kappe 5 aufgesteckt und mittels einer hier nicht gezeigten Lasche gesichert werden kann. Die Erfindung ist aber nicht nur auf Hautbehandlungsgeräte mit einer auswechselbaren Kappe 5 anwendbar, sondern auch bei Geräten, an denen die Kappe einstückig und einheitlich mit der Basis 2 ausgeführt ist.

An der hier nicht sichtbaren Rückseite der Basis 2 befindet sich eine Handelektrode 7, die in Kontakt mit der Hand steht, wenn die Basis 2 gegriffen wird. In der Basis 2 befinden sich weiterhin eine Stromquelle in Form z. B. von Batterien oder wiederaufladbaren Batterien, deren Pole mit den beiden Elektroden 4, 7 in Kontakt gebracht werden können, so dass, wenn die Hautelektrode 4 auf die Haut aufgesetzt wird, ein geschlossener Stromkreis über dem Körper der Person entsteht. Die Kappe 5 hat eine längliche Form, die sich vom Fußbereich zum Kopf hin verjüngt, wobei der Kopf typischerweise in eine halbzylindrische Kante übergeht, die über die Haut geführt werden kann.

Denkbar sind aber auch leichte Ausbeulungen der Kante oder Kappe in Form eines Kammes, wobei die Enden der Zinken aber ebenfalls von einem Halbzylinder eingehüllt werden.

Um die Creme mit einer Wirksubstanz zur Verfügung zu stellen, ist ein Ring 10 gemäß Fig. 2 vorgesehen. Dieser hat eine ovale Form mit zwei Längsabschnitten 11, die über Bögen 12 miteinander verbunden sind, wobei sich von der Außenseite des einen Längsabschnittes 11 zur Außenseite des anderen Längsabschnittes 11 ein Streifen 13 erstreckt, der als Trägermaterial für eine Creme dient.

Die innere Kontur des Ringes 10 ist an den Fußbereich der Kappe 5 angepasst, so dass der Ring 10 dort passgenau platziert werden kann, wobei sich der Streifen 13 über den Kopf der Kappe 5 erstreckt. Bei dem Trägermaterial kann es sich um einen Streifen 13 aus einem Schwamm, einem Gewebe oder einen Filz handeln.

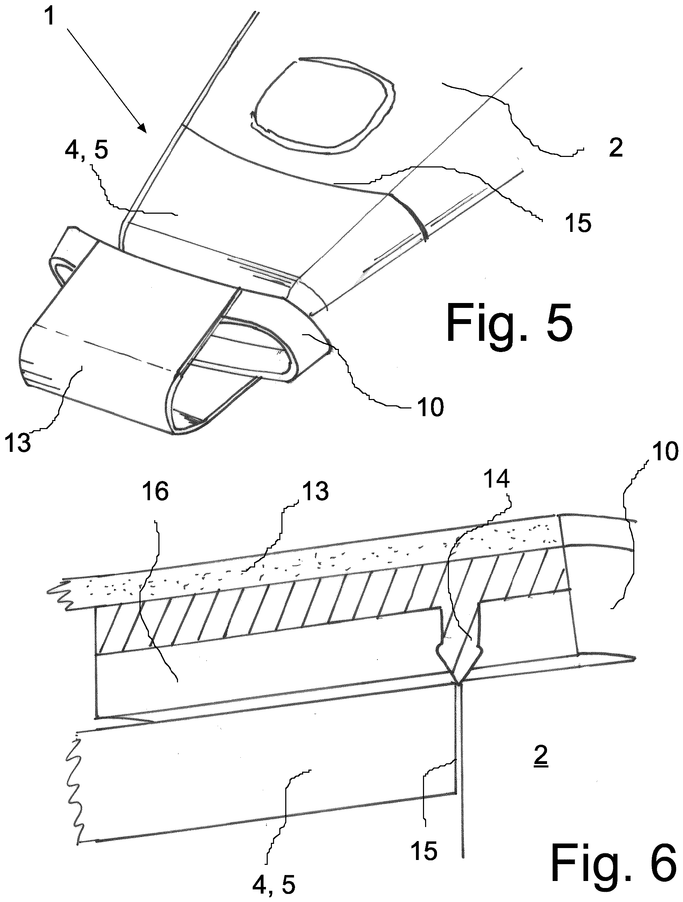

Um den Ring 10 zu sichern, sind an dessen Innenseite in der Mitte des Längsabschnittes 11 jeweils kleine Rastnasen 14 vorgesehen, die in einer spitzen Kante enden und die damit, wie die Figur 6 zeigt, in einen Spalt 15, zwischen der Basis 2 und dem Rand der Kappe 5 eingreifen.

Die Rastnasen können aber auch Haltekanten an gesondert ausgebildeten Taschen am Gerät hintergreifen.

Um aber eine ausreichende Beweglichkeit der Rastnasen 14 zu gewährleisten, sind diese jeweils am Boden einer Quernut 16 in der Innenseite des Längsabschnittes 11 des Ringes 10 eingelassen, wobei die Spitze leicht über den Rand der Nut hervorsteht.

Die Ringe 10 mit dem getränkten Streifen 13 bilden Zusatzteile, die vom Gerät gesondert, in so genannten Blistern 20 verpackt, erworben werden können.

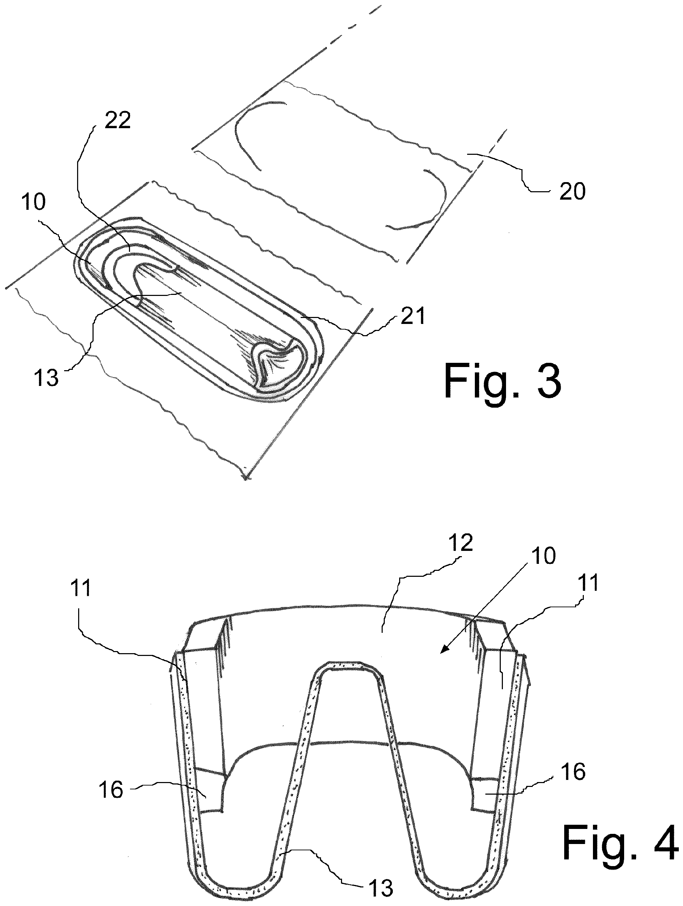

Wie die Fig. 3 zeigt, besteht ein Blister 20 aus einer Schale 21 mit einer umlaufenden Vertiefung 22, in die der Ring 10 eingelegt ist, wobei entsprechend Fig. 4, die einen Querschnitt durch den Ring 10 zeigt, der Streifen 13, ausgehend von den Längsabschnitten 11 des Ringes 10, zunächst nach unten in Richtung auf den Boden der umlaufenden Vertiefung 22 gerichtet ist und sodann in einem Bogen durch den Innenbereich des Ringes 10 verläuft. Auf diese Weise wird eine kompakte Zusammenlegung erhalten, die das Blistervolumen klein hält.

Die Schale 21 ist, was hier nicht gezeigt ist, typischerweise durch eine opake oder transparente Folie geschlossen.

Vor dem Einlegen des Ringes 10 in den Blister wird dieser mit einer eine Wirksubstanz enthaltenden Creme getränkt. Die Versiegelung des Schale 21 durch eine Folie verhindert das Austrocknen des Trägermaterials.

Zur Benutzung wird die Folie entfernt, der Ring 10 an seinen Bögen 12 gefasst und - wie die Fig. 5 zeigt - auf die Kappe 5 aufgesteckt, bis die Rastnasen 14 gemäß Fig. 5 in den Spalt 15 zwischen der Kappe 5 und Basis 2 einklipsen. Dies gewährt einen ausreichenden Halt des Ringes 10 auf dem Gerät, damit der Kopf der Kappe, über den sich der Streifen spannt, über die Haut geführt werden kann, ohne dass sich der Ring 10 vom Gerät löst.

Nach der Behandlung kann der Ring 10 wieder an den Bögen 12 gefasst, von der Kappe abgezogen und anschließend entsorgt werden.

Die Ringe 10 können farblich gestaltet werden, um die Art der Wirksubstanzen der Creme in dem Trägermaterial zu kennzeichnen.

### Bezugszeichenliste

- 1: Hautbehandlungsgerät
- 2: Basis
- 3: Sockel
- 4: Hautelektrode
- 5: Kappe

- 7: Handelektrode

- 10: Ring

- 11: Längsabschnitte
- 12: Bögen
- 13: Streifen
- 14: Rastnasen
- 15: Spalt

- 16: Quernut
- 20: Blister
- 21: Schale
- 22: Vertiefung

## Patentansprüche

1. In einer Hand haltbares Gerät zur elektrisch unterstützten Hautbehandlung, aufweisend:
Eine Hautelektrode (4), die auf den zu behandelnden Hautbereich aufsetzbar ist,
eine Handelektrode (7) an der Außenseite des Gerätes, die in Kontakt zur Hand steht, wenn das Gerät vom Nutzer beim Gebrauch in der Hand gehalten wird,
eine elektrische Energiequelle in dem Gerät, deren Pole beim Betrieb des Gerätes mit den Elektroden (4, 7) in einer elektrischen Verbindung stehen,
wobei sich vor der Hautelektrode (4) ein mit einer Wirksubstanz getränktes, saugfähiges Trägermaterial befindet,
wobei das Gerät ein Zusatzteil aufweist, das aus einem Ring (10) besteht, der auf das Gerät aufsteckbar ist und an dem das mit einer Wirksubstanz getränkte, saugfähige Trägermaterial befestigt ist, **dadurch gekennzeichnet, dass** das Trägermaterial bei einem aufgesteckten Ring über die Hautelektrode (4) verläuft, und
dass der Ring in Form eines Ovals mit zwei Längsabschnitten ausgeführt ist, die über Bögen miteinander verbunden sind,
wobei das Trägermaterial an den Längsabschnitten befestigt ist.

2. Gerät nach Anspruch 1, **dadurch gekennzeichnet, dass** das Trägermaterial aus einem zwei Enden und einem dazwischen liegenden Zwischenabschnitt aufweisenden Streifen (13) besteht, dessen Enden an dem Ring (10) befestigt sind und dessen Zwischenabschnitt bei einem aufgesteckten Ring über die Hautelektrode (4) verläuft.

3. Gerät nach Anspruch 1 oder 2, **dadurch gekennzeichnet, dass** der Ring (10) wenigstens eine nach innen gerichtete Rastnase (14) aufweist, die eine Haltekante am Gerät hintergreift.

4. Gerät nach Anspruch 1, 2 oder 3, **dadurch gekennzeichnet,**
**dass** die Hautelektrode (4) die Form einer mit einer Basis verbundenen Kappe besitzt, wobei die Kappe einen basisnahen Fußbereich und einen auf die zu behandelnde Haut aufsetzbaren Kopfbereich besitzt,
**dass** die Handelektrode (7) an der Außenseite der Basis, die in Kontakt zur Hand steht, wenn das Gerät vom Nutzer beim Gebrauch in der Hand gehalten wird, ausgebildet ist,
**dass** die elektrische Energiequelle in der Basis angeordnet ist, und
**dass** das Trägermaterial bei einem aufgesteckten Ring über den Kopf der Kappe (5) verläuft.

5. Gerät nach Anspruch 4, **dadurch gekennzeichnet, dass** der Ring (10) den Fußbereich der Kappe (5) umschließt.

6. Gerät nach Anspruch 5, **dadurch gekennzeichnet, dass** der Ring (10) wenigstens eine nach innen gerichtete Rastnase (14) aufweist, die die Unterkante der Kappe (5) hintergreift.

7. Gerät nach Anspruch 6, **dadurch gekennzeichnet, dass** die Kappe (5) auf einen Sockel (3) an der Basis (2) aufgesteckt ist, dass zwischen der Unterkante der Kappe (5) und einer Stufe an der Basis (2) ein Spalt (15) verläuft, in dem die Rastnase (14) eingreift.

8. Gerät nach Anspruch 3, 6 oder 7, **dadurch gekennzeichnet, dass** der Ring (10) an der Innenseite eine Quernut (16) aufweist, an deren Boden die Rastnase (14) angeformt ist.

9. Gerät nach einem der Ansprüche 3 und 6 bis 8,
**dadurch gekennzeichnet, dass** wenigstens zwei Rastnasen (14) vorgesehen sind, die sich gegenüberliegen.

10. Gerät nach einem der vorhergehenden Ansprüche,
**dadurch gekennzeichnet, dass** das Trägermaterial die Form eines Streifens (13) hat und dass die Hautelektrode (4) einen halbzylindrischen oberen Abschluss besitzt, über den der Streifen (13) verläuft.

11. Gerät nach einem der vorhergehenden Ansprüche,
**dadurch gekennzeichnet, dass** das Trägermaterial ein saugfähiges Gewebe, ein Schwamm oder ein Filz ist.

12. Zusatzteil zu einem in einer Hand haltbaren Gerät zur elektrisch unterstützten Hautbehandlung,
wobei das Zusatzteil aus einem Ring (10) besteht, der auf das Gerät aufsteckbar ist und an dem ein mit einer Wirksubstanz getränktes, saugfähiges Trägermaterial befestigt ist, und
**dadurch gekennzeichnet, dass** der Ring in Form eines Ovals mit zwei Längsabschnitten ausgeführt ist, die über Bögen miteinander verbunden sind,
wobei das Trägermaterial an den Längsabschnitten befestigt ist.

13. Zusatzteil nach Anspruch 12, **dadurch gekennzeichnet, dass** das Trägermaterial aus einem Streifen (13) besteht, der sich von einem Längsabschnitt (11) zum anderen erstreckt.

14. Zusatzteil nach Anspruch 13, **dadurch gekennzeichnet, dass** der Ring (10) an der Innenseite der Längsabschnitte (11) jeweils mindestens eine Rastnase (14) aufweist, die über die innere Mantelfläche des Ringes (10) hervorsteht.

15. Zusatzteil nach Anspruch 14, **dadurch gekennzeichnet, dass** die Rastnasen (14) in einer Quernut (16) am Ring (10) angeordnet sind.

16. Zusatzteil nach einem der Ansprüche 12 bis 15,
**dadurch gekennzeichnet, dass** es in einer hermetisch geschlossenen Verpackung aufbewahrt ist.

17. Blister mit einem Zusatzteil nach einem der vorhergehenden Ansprüche 12 bis 16, **dadurch gekennzeichnet, dass** der Blister (20) aus einer Schale (21) mit einer Folienabdeckung besteht, wobei sich in der Schale (21) eine umlaufende Vertiefung (22) zur Aufnahme des Ringes (10) befindet und wobei der Streifen (13) zum Schalenboden weist und sein mittlerer Teil in einem Bogen durch den Ring (10) verläuft.

## Claims

1. Device for electrically assisted skin treatment which can be held in a hand, comprising
A skin electrode (4) which can be placed on the skin area to be treated,
a hand electrode (7) on the outside of the device, which is in contact with the hand when the device is held in the hand by the user during use,
an electrical energy source in the device, the poles of which are in electrical contact with the electrodes (4, 7) during operation of the device,
wherein an absorbent carrier material impregnated with an active substance is located in front of the skin electrode (4),
wherein the device has an additional part which consists of a ring (10) which can be plugged onto the device and to which the absorbent carrier material impregnated with an active substance is attached,
**characterised in that** the carrier material extends over the skin electrode (4) when the ring is plugged on, and
**in that** the ring is designed in the form of an oval with two longitudinal sections which are connected to one another via arcs,
wherein the carrier material is attached to the longitudinal sections.

2. Device according to claim 1, **characterised in that** the carrier material consists of a strip (13) having two ends and an intermediate section between them, the ends of which are attached to the ring (10) and the intermediate section of which extends over the skin electrode (4) when a ring is plugged on.

3. Device according to claim 1 or 2, **characterised in that** the ring (10) has at least one inwardly directed latching lug (14) which engages behind a retaining edge on the device.

4. Device according to claim 1, 2 or 3, **characterised**
**in that** the skin electrode (4) has the form of a cap connected to a base, the cap having a foot area close to the base and a head area which can be placed on the skin to be treated,
**in that** the hand electrode (7) is formed on the outside of the base, which is in contact with the hand when the device is held in the hand by the user during use,
**in that** the electrical energy source is arranged in the base, and
**in that** the carrier material extends over the head of the cap (5) when a ring is plugged on.

5. Device according to claim 4, **characterised in that** the ring (10) encloses the base area of the cap (5).

6. Device according to claim 5, **characterised in that** the ring (10) has at least one inwardly directed latching lug (14) which engages behind the lower edge of the cap (5).

7. Device according to claim 6, **characterised in that** the cap (5) is fitted onto a socket (3) on the base (2), **in that** a gap (15) extends between the lower edge of the cap (5) and a step on the base (2), in which gap the latching lug (14) engages.

8. Device according to claim 3, 6 or 7, **characterised in that** the ring (10) has a transverse groove (16) on the inside, at the bottom of which the latching lug (14) is formed.

9. Device according to one of claims 3 and 6 to 8, **characterised in that** at least two latching lugs (14) are provided which are opposite each other.

10. Device according to any of the preceding claims, **characterised in that** the carrier material has the form of a strip (13) and **in that** the skin electrode (4) has a semi-cylindrical upper end over which the strip (13) extends.

11. Device according to any of the preceding claims, **characterised in that** the carrier material is an absorbent fabric, a sponge or a felt.

12. Additional part for a device for electrically assisted skin treatment which can be held in a hand, wherein the additional part consists of a ring (10) which can be plugged on the device and to which an absorbent carrier material impregnated with an active substance is attached, and
**characterised in that** the ring is designed in the form of an oval with two longitudinal sections which are connected to one another via arches,
wherein the carrier material is attached to the longitudinal sections.

13. Additional part according to claim 12, **characterised in that** the carrier material consists of a strip (13) which extends from one longitudinal section (11) to the other.

14. Additional part according to claim 13, **characterised in that** the ring (10) has at least one latching lug (14) on the inside of each of the longitudinal sections (11), which latching lug projects beyond the inner circumferential surface of the ring (10).

15. Additional part according to claim 14, **characterised in that** the latching lug (14) are arranged in a transverse groove (16) on the ring (10).

16. Additional part according to any of claims 12 to 15, **characterised in that** it is stored in a hermetically sealed package.

17. Blister with an additional part according to any of the preceding claims 12 to 16, **characterised in that** the blister (20) consists of a tray (21) with a foil cover, wherein a circumferential recess (22) for receiving the ring (10) is located in the tray (21) and wherein the strip (13) faces the bottom of the tray and its central part extends in an arc through the ring (10).

## Revendications

1. Appareil de traitement de la peau à assistance électrique, pouvant être tenu dans une main, comprenant :
une électrode cutanée (4) qui peut être placée sur la zone de peau à traiter,
une électrode manuelle (7) située à l'extérieur de l'appareil, qui est en contact avec la main lorsque l'appareil est tenu à la main par l'utilisateur lors de l'utilisation,
une source d'énergie électrique dans l'appareil, dont les pôles sont en liaison électrique avec les électrodes (4, 7) pendant le fonctionnement de l'appareil,
un matériau porteur absorbant imprégné d'une substance active se trouvant devant l'électrode cutanée (4),
l'appareil présentant une pièce supplémentaire qui se compose d'un anneau (10) qui peut être enfiché sur l'appareil et sur lequel est fixé le matériau porteur absorbant imprégné d'une substance active,
**caractérisé en ce que** le matériau porteur s'étend sur l'électrode cutanée (4) lorsqu'un anneau est enfiché, et
**en ce que** l'anneau est réalisé sous la forme d'un ovale avec deux sections longitudinales qui sont reliées entre elles par des arcs,
le matériau porteur étant fixé aux sections longitudinales.

2. Appareil selon la revendication 1, **caractérisé en ce que** le matériau porteur est constitué d'une bande (13) présentant deux extrémités et une section intermédiaire située entre celles-ci, dont les extrémités sont fixées à l'anneau (10) et dont la section intermédiaire s'étend sur l'électrode cutanée (4) lorsque l'anneau est enfiché.

3. Appareil selon la revendication 1 ou 2, **caractérisé en ce que** l'anneau (10) présente au moins un ergot d'encliquetage (14) orienté vers l'intérieur, qui s'engage derrière une arête de retenue sur l'appareil.

4. Appareil selon la revendication 1, 2 ou 3, **caractérisé**
**en ce que** l'électrode cutanée (4) a la forme d'un capuchon relié à une base, le capuchon ayant une zone de pied proche de la base et une zone de tête pouvant être posée sur la peau à traiter,
**en ce que** l'électrode manuelle (7) est formée sur le côté extérieur de la base qui est en contact avec la main lorsque l'appareil est tenu à la main par l'utilisateur lors de l'utilisation,
**en ce que** la source d'énergie électrique est disposée dans la base, et
**en ce que** le matériau porteur s'étend au-dessus de la tête du capuchon (5) lorsqu'un anneau est enfiché.

5. Appareil selon la revendication 4, **caractérisé en ce que** l'anneau (10) entoure la zone de pied du capuchon (5).

6. Appareil selon la revendication 5, **caractérisé en ce que** l'anneau (10) présente au moins un ergot d'encliquetage (14) orienté vers l'intérieur, qui s'engage derrière le bord inférieur du capuchon (5).

7. Appareil selon la revendication 6, **caractérisé en ce que** le capuchon (5) est emboîté sur un socle (3) sur la base (2), **en ce qu'**entre le bord inférieur du capuchon (5) et une marche sur la base (2) s'étend une fente (15) dans laquelle s'engage l'ergot d'encliquetage (14).

8. Appareil selon la revendication 3, 6 ou 7, **caractérisé en ce que** l'anneau (10) présente sur le côté intérieur une rainure transversale (16) au fond de laquelle est formé l'ergot d'encliquetage (14).

9. Appareil selon l'une des revendications 3 et 6 à 8, **caractérisé en ce qu'**il est prévu au moins deux ergots d'encliquetage (14) qui se font face.

10. Appareil selon l'une des revendications précédentes, **caractérisé en ce que** le matériau porteur a la forme d'une bande (13) et **en ce que** l'électrode cutanée (4) possède une terminaison supérieure semi-cylindrique sur laquelle s'étend la bande (13).

11. Appareil selon l'une **quelconque** des revendications précédentes, **caractérisé en ce que** le matériau porteur est un tissu absorbant, une éponge ou un feutre.

12. Pièce supplémentaire d'un appareil de traitement de la peau à assistance électrique pouvant être tenu dans une main,
la pièce supplémentaire étant constituée d'un anneau (10) qui peut être enfiché sur l'appareil et auquel est fixé un matériau porteur absorbant imprégné d'une substance active, et
**caractérisée en ce que** l'anneau est réalisé sous la forme d'un ovale avec deux sections longitudinales qui sont reliées entre elles par des arcs,
le matériau porteur étant fixé aux sections longitudinales.

13. Piècesupplémentaire selon la revendication 12, **caractérisée en ce que** le matériau porteur est constitué d'une bande (13) qui s'étend d'une section longitudinale (11) à l'autre.

14. Pièce supplémentaire selon la revendication 13, **caractérisée en ce que** l'anneau (10) présente sur le côté intérieur des sections longitudinales (11) respectivement au moins un ergot d'encliquetage (14) qui fait saillie sur la surface d'enveloppe intérieure de l'anneau (10).

15. Pièce supplémentaire selon la revendication 14, **caractérisée en ce que** les ergots d'encliquetage (14) sont disposés dans une rainure transversale (16) sur l'anneau (10).

16. Pièce supplémentaire selon l'une des revendications 12 à 15, **caractérisée en ce qu'**elle est conservée dans un emballage hermétiquement fermé.

17. Blister avec une pièce supplémentaire selon l'une des revendications 12 à 16 précédentes, **caractérisé en ce que** le blister (20) est constitué d'une coque (21) avec un recouvrement en film, un creux périphérique (22) se trouvant dans la coque (21) pour recevoir l'anneau (10) et la bande (13) étant dirigée vers le fond de la coque et sa partie centrale s'étendant en arc à travers l'anneau (10).
